# EUROPEAN PATENT APPLICATION

(11) **EP 2 466 874 A2**
(43) Date of publication of application: **20.06.2012**
(21) Application number: 11187516.7
(22) Date of filing: 02.11.2011
(51) Int. Cl.: H04N 5/235

(54) **Image processing device**

(30) Priority: 16.12.2010 JP 2010280629
(71) Applicant: Fujifilm Corporation, Minato-ku Tokyo (JP)
(72) Inventor: KAKU, Toshihiko, Kanagawa (JP)
(74) Representative: Klunker . Schmitt-Nilson . Hirsch

(57) **Abstract**

The image processing device includes an image acquisition unit for acquiring a normal light observation image captured with white light and an original special light observation image captured simultaneously with the normal light observation image using predetermined narrowband light, and an image processing unit for subjecting the original special light observation image acquired by the image acquisition unit to predetermined processing to generate a special light observation image and providing information of the special light observation image to the normal light observation image.

## Description

### BACKGROUND OF THE INVENTION

The present invention relates to an image processing device capable of an endoscopic observation making use of the characteristics of both of a normal light observation image obtained with white light and a special light observation image obtained with specific narrowband light.

In recent years, use is made of an endoscope system capable of so-called special light observation in which a mucosal tissue of a living body is irradiated with specific narrow wavelength band light (narrowband light) as observation light to obtain tissue information at a desired depth of the tissue of the living body.

The special light observation can simply visualize biological information which is not obtainable from the normal observation image, such as the microstructure in the superficial layer of a neovascular vessel formed in a mucosal layer or a submucosal layer, or an enhanced lesion. For example when a cancer lesion is to be observed, a mucosal tissue is irradiated with blue (B) narrowband light suitable to the observation of the tissue in the superficial layer and green (G) narrowband light suitable to the observation of the tissue in the middle and deep layers, whereby the state of the microvessels and microstructure in the superficial layer of the tissue can be more precisely observed and the lesion can be therefore more correctly diagnosed.

The endoscope system as described in JP 3559755 B is known as an endoscope system having the functions of normal light observation and special light observation.

The endoscope system uses a light source device which includes a light source for emitting white light and a rotary filter having an R filter for converting white light into red (R) light, a G filter for converting white light into green (G) light and a B filter for converting white light into blue (B) light, and an endoscope which captures images with an imaging device for measuring without separation of incident light. The endoscope system captures images in a so-called frame sequential manner.

The endoscope system described in JP 3559755 B uses the rotary filter of a double structure including a first set of outside filters and a second set of inside filters.

The first set of outside filters are filters for use in the normal light observation which have such spectral characteristics that the wavelength ranges of the respective colors overlap each other. On the other hand, the second set of inside filters are filters for use in the special light observation which have such discrete narrowband spectral characteristics that the wavelength ranges of the respective colors are separated from each other.

Therefore, this endoscope system is capable of both of the normal light observation and the special light observation by shifting the rotational axis of the rotary filter so that the first set of filters may act on the optical path when the normal light observation is performed and the second set of filters may act on the optical path when the special light observation is performed.

However, switching between the normal light observation and the special light observation in this endoscope system requires changeover of the rotary filter. Therefore, both of a normal light observation image and a special light observation image cannot be simultaneously observed in the same biological tissue.

Under the circumstances, various proposals have been made to enable a normal light observation and a special light observation to be performed simultaneously.

For example, JP 2004-321244 A describes an endoscope system (electronic endoscope system) which involves alternately capturing and storing normal light images and special light images at predetermined timings, reading out images at predetermined timings and subjecting the images to mutually different processing to acquire a normal light observation image and a special light observation image (substantially) simultaneously.

In this endoscope system, the normal light observation image and the special light observation image are observed simultaneously by displaying the two images acquired at the same period one by one, side by side on one screen or in an overlapping manner (composition of two images).

JP 2008-43604 A describes an endoscope system which processes a color image having R, G and B images captured in limited bands with a low-pass filter or a band-pass filter to generate image signals of a normal light observation image and those of a special light observation image thereby acquiring the normal light observation image and the special light observation image simultaneously.

In this endoscope system as well, the thus acquired normal light observation image and special light observation image are observed simultaneously by displaying the two images one by one or side by side on one screen.

### SUMMARY OF THE INVENTIION

The endoscope systems described in JP 2004-321244 A and JP 2008-43604 A can simultaneously display and observe a special light image and a normal light image in the same biological tissue.

However, according to the systems described in these documents, the special light observation image must be compared with the normal light observation image, which requires time and effort, and an endoscopic image making full of the characteristics of both of the normal light observation image and the special light observation image (e.g., the advantage of the special light observation image that microvessels in the superficial layer are easily observed and the advantage of the normal light observation image that the image is light and the structure and state of the whole image region including the periphery of the site to be observed can be easily recognized) is not obtained yet.

An object of the present invention is to solve the foregoing prior art problems and to provide an image processing device capable of acquiring a normal light observation image and a special light observation image captured with an endoscope simultaneously or substantially simultaneously and displaying an image making full use of the characteristics of the normal light observation image and the special light observation image.

In order to achieve the above object, the present invention provides an image processing device comprising: an image acquisition unit for acquiring a normal light observation image captured by an endoscope using white light as observation light and an original special light observation image captured by the endoscope simultaneously with the normal light observation image using predetermined narrowband light as the observation light; and an image processing unit for subjecting the original special light observation image acquired by the image acquisition unit to predetermined processing to generate a special light observation image and providing information of the special light observation image to the normal light observation image.

The image processing unit preferably performs frequency processing on the original special light observation image as the predetermined processing.

The image processing unit preferably has at least one function selected from a function of processing the original special light observation image with a high-pass filter, a function of processing the original special light observation image with a band-pass filter, and a function of processing the original special light observation image with a low-pass filter.

Preferably, the image processing device further comprises a filter selector for selecting a filter to be used in the frequency processing of the original special light observation image in the image processing unit, the image processing unit has different filters for use in the frequency processing of the original special light observation image and a filter selected by the filter selector is used to perform the frequency processing on the original special light observation image. Preferably, the image processing unit prepares a first image obtained by performing the frequency processing on the original special light observation image with a first filter and a second image obtained by performing the frequency processing on the original special light observation image with a different filter from the first filter, and information of an image obtained by compositing the first image with the second image or information obtained by subtracting one of the first image and the second image from the other is provided to the normal light observation image.

The image processing unit preferably extracts a predetermined color component from the original special light observation image as the predetermined processing. The image processing unit preferably extracts a portion having a predetermined contrast from the original special light observation image as the predetermined processing. The image processing unit preferably extracts a portion having a predetermined structure as the predetermined processing.

The image processing unit preferably checks the special light observation image after the predetermined processing for a degree of denseness and further extracts a region where the degree of denseness exceeds a predetermined value.

Preferably, the image processing device further comprises a processing selector for selecting enhancement or suppression of the special light observation image generated by processing the original special light observation image, and the image processing unit enhances or suppresses the special light observation image generated by processing the original special light observation image in accordance with a selection made by the processing selector. The image processing unit preferably checks the special light observation image after the predetermined processing for a degree of denseness and performs at least one processing selected from enhancement of a region where the degree of denseness exceeds a predetermined value and suppression of a region where the degree of denseness is up to the predetermined value.

The image processing device of the invention acquires a normal light observation image captured with an endoscope using white light as observation light and a special light observation image captured with the endoscope (substantially) at the same time at the same position as for the normal light image and using specific narrowband light as observation light, processes the special light observation image, and generates an image in which information of the processed special light observation image is provided to the normal light observation image by image composition or other processing.

Therefore, according to the image processing device of the invention, an image in which microvessels in the superficial layer and thick blood vessels in the middle and deep layers are provided to the normal light observation image, an image in which the microvessels in the superficial layer are only provided to the normal light observation image, and an image in which the thick blood vessels in the middle and deep layers are only provided to the normal light observation image can be generated by extracting the capillaries in the superficial layer and the thick blood vessels in the middle and deep layers from the special light observation image through, for example, frequency processing.

Accordingly, the invention is capable of using a special light observation image and a normal light observation image captured simultaneously at the same position to generate and display an image making full use of the characteristics of the normal light observation image and the special light observation image, the image being obtained by providing an image of a remarkable site captured in the special light observation to the normal light image serving as the base which is light and in which the structure and state of the whole image region including the depth can be easily recognized.

### BRIEF DESCRIPTION OF THE DRAWINGS

FIG. 1 is a conceptual diagram showing an embodiment of an endoscope system to which an image processing device of the invention is applied.
FIG. 2 is a conceptual block diagram showing the configuration of the endoscope system shown in FIG. 1.
FIGS. 3A and 3B are conceptual diagrams showing exemplary rotary filters used in the endoscope system shown in FIG. 1.
FIG. 4 is a conceptual block diagram showing a processor of the endoscope system shown in FIG. 1.
FIG. 5 is a conceptual block diagram showing the configuration of another embodiment of the endoscope system according to the invention.
FIG. 6 is a conceptual block diagram showing the configuration of still another embodiment of the endoscope system according to the invention.
FIG. 7 is a conceptual diagram showing an exemplary spectrometric profile of observation light in the endoscope system shown in FIG. 6.

### DETAILED DESCRIPTION OF THE INVENTION

On the following pages, the image processing device of the invention is described in detail with reference to the preferred embodiments illustrated in the accompanying drawings.

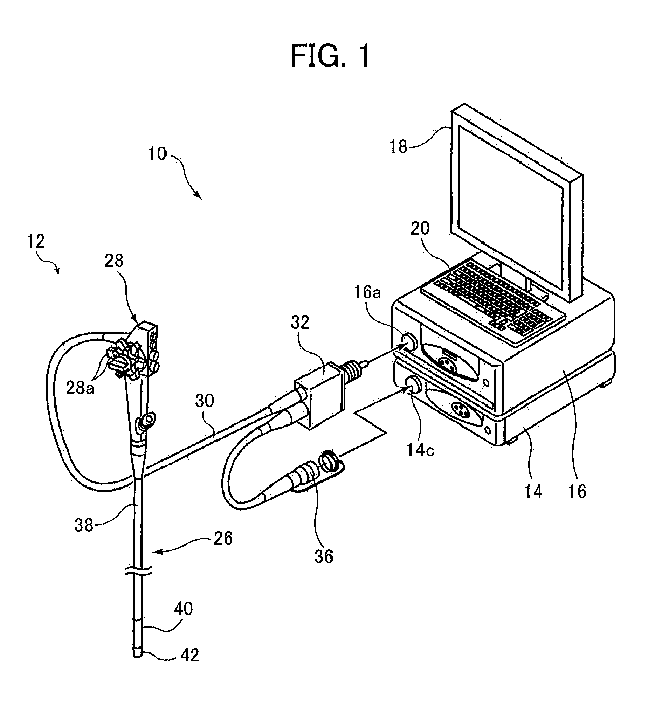

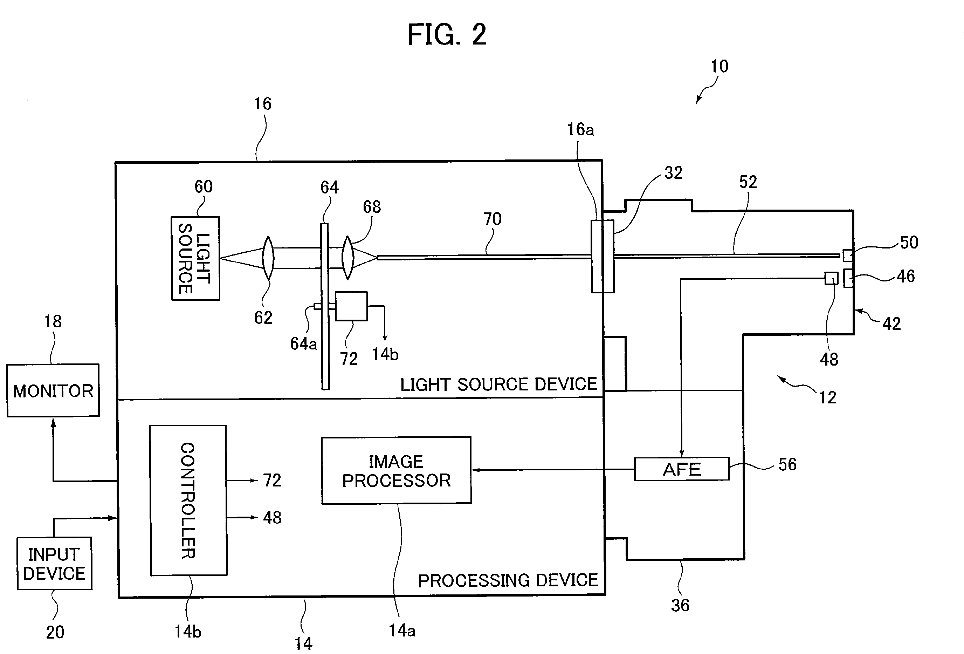

FIG. 1 is a schematic perspective view showing an exemplary endoscope system which uses the image processing device of the invention and FIG. 2 conceptually shows the configuration of the endoscope system shown in FIG. 1.

The illustrated endoscope system 10 includes, for example, an endoscope 12, a processing device 14 for processing an image captured by the endoscope 12 and a light source device 16 for supplying observation light (illumination light) for use in observation and image capture using the endoscope 12.

The processing device 14 includes a monitor 18 for displaying an image captured by the endoscope and an input device 20 for inputting various instructions (the processing device 14 is connected to the monitor 18 and the input device 20). The processing device 14 may further include a printer (recording unit) for outputting an image captured by the endoscope as a hard copy.

In the illustrated endoscope system 10, the processing device 14 makes up the image processing device of the invention.

As shown in FIG. 2, the endoscope 12 is an electronic endoscope which photoelectrically captures an image using an imaging device such as a CCD sensor 48. As in a common endoscope, the endoscope 12 includes an insertion section 26, an operating section 28, a universal cord 30, a connector 32 and a video connector 36.

During the observation (diagnosis), the endoscope 12 is used with the video connector 36 and the connector 32 connected to a connecting portion 14c of the processing device 14 and a connecting portion 16a of the light source device 16, respectively. As in a common endoscope, the connector 32 is connected to a suction means and an air supply means for the suction from and the air supply to the site to be observed, and a water supply means for the water injection on the site to be observed.

As in a common endoscope, the insertion section 26 of the endoscope 12 includes a long flexible portion 38 on the proximal side, a distal scope portion (endoscope distal portion) 42 provided with the CCD sensor 48 and the like, and a bending portion (angle portion) 40 located between the flexible portion 38 and the scope portion 42. The operating section 28 includes manipulation knobs 28a for bending the bending portion 40.

As schematically shown in FIG. 2, the scope portion 42 is provided with an imaging lens 46, the CCD sensor 48, an illumination lens 50, an optical fiber 52 and a cover glass (not shown) for protecting the lenses and the like.

Although not shown, the endoscope 12 is also provided with a forceps channel and a forceps port for inserting various treatment tools such as a forceps, and air supply/water supply channels and air supply/water supply ports for use in suction, air supply and water supply.

The forceps channel extends through the bending portion 40 and the flexible portion 38 to communicate with a forceps insertion port provided in the operating section 28, and the air supply/water supply channels extend through the bending portion 40, the flexible portion 38, the operating section 28 and the universal cord 30 to communicate with connecting portions with the suction means, the air supply means and the water supply means in the connector 32.

The optical fiber 52 extends through the bending portion 40, the flexible portion 38, the operating section 28 and the universal cord 30 and terminated by the connector 32 which is connected to the light source device 16.

Observation light emitted from the light source device 16 to be described later enters the optical fiber 52 through the connector 32 and propagates through the optical fiber 52. In the scope portion 42, the light enters the illumination lens 50 from the distal end of the optical fiber 52 and passes through the illumination lens 50 to be irradiated on an observation site.

The observation site having received the observation light is imaged through the imaging lens 46 on the light-receiving surface of the CCD sensor 48.

In the illustrated endoscope system 10, the CCD sensor 48 used in the endoscope 12 is a so-called monochrome CCD sensor which captures light incident on the light-receiving surface without separating it into bands of R (red), B (blue) and G (green) colors. The CCD sensor 48 images the observation site by a so-called frame sequential process using R light, G light, B light and narrowband B light supplied from the light source device 16 to be described later as the observation light.

In the practice of the invention, the imaging device is not limited to the CCD sensor 48 and various known imaging devices such as a CMOS image sensor may be used.

Output signals from the CCD sensor 48 are sent on signal lines from the scope portion 42 to the video connector 36 through the bending portion 40, the flexible portion 38, the operating section 28, the universal cord 30 and the connector 32.

In the illustrated embodiment, an AFE (Analog Front End) board 56 is disposed in the video connector 36.

The AFE board 56 includes, for example, a correlated double sampling circuit, an amplifier (automatic gain control circuit) and an A/D converter. In the AFE board 56, the output signals from the CCD sensor 48 are subjected to noise removal by correlated double sampling, amplification in the amplifier and conversion of analog signals into digital signals in the A/D converter, and then outputted to the processing device 14 (more specifically to a DSP 76 to be described later) as digital image signals.

In the endoscope system of the invention, these processing steps may be performed in the connector 32 or the processing device 14 instead of the video connector 36.

As described above, the connector 32 of the endoscope 12 in the endoscope system 10 is connected to the connecting portion 16a of the light source device 16.

The light source device 16 supplies the endoscope 12 with observation light for the internal observation of a living body. As described above, the observation light supplied from the light source device 16 toward the endoscope 12 enters the optical fiber 52 through the connector 32 and propagates therethrough to be irradiated on the observation site through the scope portion 42.

As schematically shown in FIG. 2, the light source device 16 of the endoscope system 10 includes a light source 60, a collimator lens 62, a rotary filter 64, a condenser lens 68, an optical fiber 70, a rotary drive source 72 and the connecting portion 16a.

The light source 60 is one for emitting light used for the observation.

In the light source device 16 of the illustrated endoscope system 10, various light sources capable of emitting white light that are used in known endoscope systems may be employed for the light source 60, as exemplified by a xenon lamp and a white LED.

White light emitted from the light source 60 is collimated by the collimator lens 62 into parallel light, which enters the rotary filter (filter turret) 64.

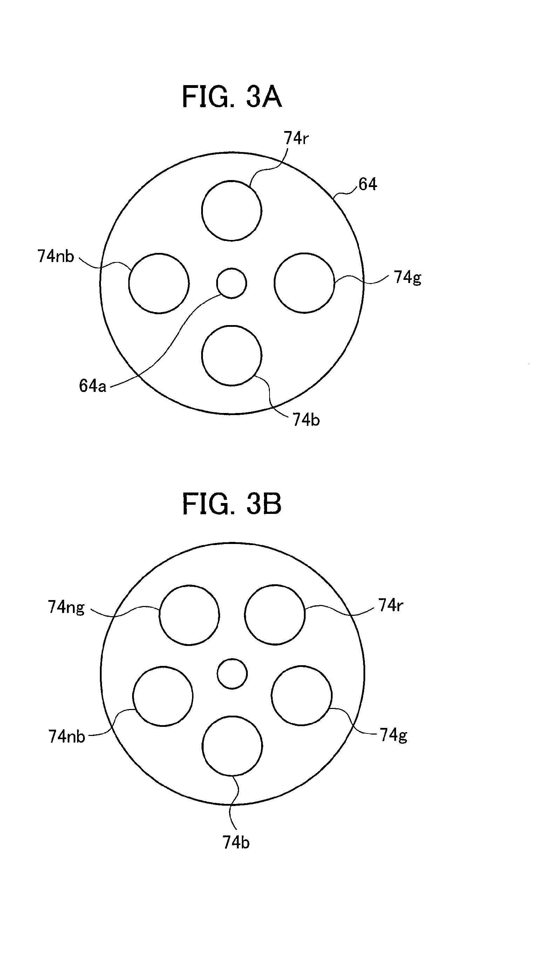

FIG. 3A is a conceptual diagram showing the rotary filter 64. The rotary filter 64 includes four filters such as an R filter 74r for converting white light into R light, a G filter 74g for converting white light into G light, a B filter 74b for converting white light into B light and a narrowband B filter 74nb for converting white light into narrowband B light.

The R, G and B filters are known color filters that may be used to take a color image by separating light into components of three primary colors of red, green and blue.

The narrowband B light is, for example, light with a wavelength of 380 to 480 nm and a bandwidth in spectral distribution of 75 nm or less. Specifically various types of narrowband B light used in so-called special light observation (narrowband light observation) in the endoscope, more specifically light with a wavelength of 390 to 445 nm and particularly light with a wavelength of 400±10 nm and a central wavelength of 405 nm may be used.

Various filters which can convert white light into such narrowband B light (hereinafter referred to as "narrowband B light") may be used for the narrowband B filter 74nb.

The rotary drive source 72 which is controlled for the drive (rotation) by the controller 14b of the processing device 14 to be described later causes the rotary filter 64 to rotate about a rotary shaft 64a to sequentially insert the filters in the optical path of light having passed through the collimator lens 62.

In other words, white light emitted from the light source 60 is separated by the inserted filters of the rotary filter 64 into R light, G light, B light and narrowband B light in a time-sharing manner.

Light having passed through the filters of the rotary filter 64 is condensed by the condenser lens 68 and the condensed light impinges on the incident end face of the optical fiber 70.

The light having entered the optical fiber 70 propagates therethrough and passes through the connecting portion 16a and the connector 32 of the endoscope 12 to enter the optical fiber 52 of the endoscope 12. Then, the light propagates through the optical fiber 52 and is irradiated from the scope portion 42 on the observation site as observation light and the CCD sensor 48 captures an image of the observation site.

As described above, the light source device 16 uses the rotary filter 64 to separate white light from the light source 60 into R light, G light, B light and narrowband B light in a time sharing manner and supplies them as the observation light. Therefore, the R light, G light, B light and narrowband B light are sequentially and repeatedly supplied to the endoscope 12 and are then irradiated from the scope portion 42 on the observation site.

Under the control of the controller 14b, the CCD sensor 48 sequentially captures (samples) images for the light of the respective colors in synchronization with the rotary filter 64 (at a timing suitable for the rotation of the rotary filter 64). That is, the monochrome CCD sensor 48 captures an R image, a G image, a B image and a narrowband B image in a frame sequential manner upon the irradiation with the R light, G light, B light and narrowband B light, respectively.

The processing device 14 of the endoscope system 10 obtains from the R image, G image and B image a normal light observation image using white light as the observation light and from the narrowband B image and G image a special light observation image using special light as the observation light. In other words, the endoscope system 10 can obtain a normal light observation image and a special light observation image which are taken simultaneously or substantially simultaneously. This point will be described in detail later.

In the light source device 16 used in the illustrated endoscope system 10, the rotary filter 64 is not limited to a type having four filters as shown in FIG. 3A.

For example, a rotary filter having five filters as shown in FIG. 3B may also be advantageously used which includes a narrowband G filter 74ng for converting white light into narrowband G light in addition to the R filter 74r, the G filter 74g, the B filter 74b and the narrowband B filter 74nb.

In this case, the processing device 14 obtains from the R image, G image and B image a normal light observation image using white light as the observation light and from the narrowband B image and narrowband G image obtained from the narrowband G light a special light observation image using special light as the observation light.

The narrowband G light is, for example, light with a wavelength of 480 to 580 nm and a bandwidth in spectral distribution of 75 nm or less. Specifically various types of narrowband G light used in so-called special light observation and more specifically light with a wavelength of 530 to 550 nm may be used.

Various filters which can convert white light into such narrowband G light (hereinafter referred to as "narrowband G light") may be used for the narrowband G filter 74ng.

The observation site having received the observation light from the light source device 16 is imaged by the CCD sensor 48.

As described above, the image captured by the CCD sensor 48 (output signals from the CCD sensor 48) is subjected to processing such as A/D conversion in the AFE board 56 and supplied to the processing device 14 as digital image signals (image data/image information).

The processing device 14 subjects the image signals supplied (outputted) from the endoscope 12 (the image signals are also referred to simply as an "image") to predetermined processing so that the monitor 18 displays them as an image captured by the endoscope 12 and also controls the endoscope system 10. The processing device 14 includes an image processor 14a and a controller 14b for controlling the whole of the endoscope system 10 including the processing device 14.

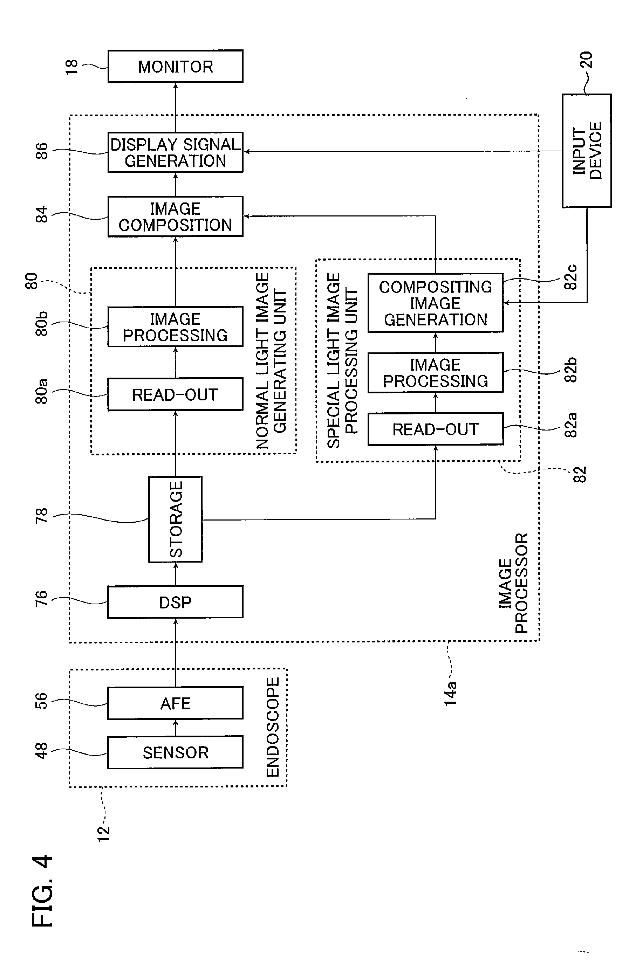

FIG. 4 is a conceptual block diagram showing the image processor 14a of the processing device 14.

As shown in FIG. 4, the image processor 14a includes the DSP 76, a storage unit 78, a normal light image generating unit 80, a special light image processing unit 82, an image composition unit 84 and a display signal generating unit 86.

In the processing device 14, images (R image, G image, B image and narrowband B image) from the endoscope 12 are supplied to the DSP 76.

The DSP 76 is a known type of DSP (Digital Signal Processor), where the supplied images are subjected to predetermined processing steps such as gamma correction and color correction. The processed images are then stored in a predetermined region of the storage unit (memory) 78.

Once the images are stored in the storage unit 78, the normal light image generating unit 80 reads out the R, G and B images from the storage unit 78 to generate a normal light observation image. The special light image processing unit 82 reads out the narrowband B image and the G image from the storage unit 78 to generate a special light observation image, from which an image for composition is generated.

As described above, the R, G, B and narrowband B images are images simultaneously taken by rotating the rotary filter 64 and therefore the normal light observation image and the special light observation image are simultaneously taken images.

The normal light image generating unit 80 includes a read-out section 80a and an image processing section 80b.

As described above, the read-out section 80a is a section in which the R, G and B images forming the normal light observation image are read out from the images of the respective colors stored in the storage unit 78 and supplied to the image processing section 80b.

The image processing section 80b subjects the R, G and B images read out by the read-out section 80a to processing with a 3x3 matrix, gradation conversion, processing with a three-dimensional LUT or other color conversion processing; color enhancement for giving a color difference between a blood vessel and a mucous membrane on the screen by enhancing in a direction in which the color difference between the blood vessel and the mucous membrane is to be more accentuated than the average colors of the image so that the blood vessel can be more easily seen; and image structure enhancement such as sharpening and edge enhancement, and supplies the processed image to the image composition unit 84 as the normal light observation image.

On the other hand, the special light image processing unit 82 includes a read-out section 82a, an image processing section 82b and a compositing image generating section 82c.

As described above, the read-out section 82a is a section in which the narrowband B image and the G image forming the special light observation image is read out from the images of the respective colors stored in the storage unit 78 and supplies them to the image processing section 82b.

When the rotary filter of the light source device 16 has five filters including the narrowband G filter 74ng as shown in FIG. 3B, five images including the R image, G image, B image, narrowband B image and narrowband G image are supplied from the endoscope 12.

In this case, the read-out section 82a does not read out the G image. The read-out section 82a reads out the narrowband B image and the narrowband G image from the storage unit 78 as the images for forming the special light observation image and supplies them to the image processing section 82b. The following processing steps in the special light image processing unit 82 are performed on the narrowband G image instead of the G image.

The image processing section 82b is a section in which the narrowband B image and the G image are processed to obtain the special light observation image.

On the displayed image (image outputted from the processing device 14), three sub-pixels of R, G and B make up one pixel. In the illustrated embodiment, however, the special light observation image is only obtained from the narrowband B image and the G image. Therefore, the image processing section 82b first allocates the G image to R pixels corresponding to the display and the narrowband B image to G pixels and B pixels corresponding to the display to form an image in which three sub-pixels of R, G and B corresponding to the display make up one pixel.

The image allocation may be optionally preceded by image processing or correction such as multiplication of an image by a predetermined coefficient.

The image processing section 82b subjects the images allocated to the R, G and B pixels to processing with a 3x3 matrix, gradation conversion, processing with a three-dimensional LUT or other color conversion processing; color enhancement for giving a color difference between a blood vessel and a mucous membrane on the screen by enhancing in a direction in which the color difference between the blood vessel and the mucous membrane is to be more accentuated than the average colors of the image so that the blood vessel can be more easily seen; and image structure enhancement such as sharpening and edge enhancement, and supplies the processed image to the compositing image generating section 82c as the special light observation image.

The compositing image generating section 82c is a section in which the special light observation image formed in the image processing section 82b is subjected to predetermined processing to generate a compositing image for compositing with the normal light image generated in the normal light image generating unit 80 (a compositing image for providing information of the special light observation image to the normal light observation image) and supplies the compositing image to the image composition unit 84.

In the illustrated embodiment, the compositing image generating section 82c first subjects the special light observation image generated by the image processing section 82b to frequency processing using, for example, a low-pass filter (LPF), a band-pass filter (BPF) or a high-pass filter (HPF).

The frequency band in which processing is made using these filters may be appropriately set according to the site to be observed and the image to be obtained.

As is well known, according to the special light observation using narrowband B light and G light (preferably narrowband G light), microvessels in the superficial layer of the mucous membrane and thick blood vessels in the middle and deep layers of the mucous membrane can be imaged (information on these blood vessels can be obtained).

Therefore, a special light observation image in which microvessels in the superficial layer of the mucous membrane are extracted (that is, blood vessels in the middle and deep layers of the mucous membrane are removed) is obtained from an original special light observation image by processing the original special light observation image with an HPF or a BPF which passes high frequencies within a predetermined range. In the following description, for the sake of convenience, the original special light observation image (special light observation image obtained from the images captured by the CCD sensor 48) is referred to below as "original special light image" and the special light observation image obtained by processing the original special light image with a filter (color extraction) is referred to below as "processed special light image."

On the other hand, a processed special light image in which thick blood vessels in the middle and deep layers of the mucous membrane are extracted (that is, microvessels in the superficial layer of the mucous membrane are removed) is obtained from the original special light image by processing the original special light image with an LPF or a BPF which passes low frequencies within a predetermined frequency band.

By processing the original special light image with different filters to generate a plurality of processed special light images and compositing these images, a processed special light image in which specific sites extracted by the filters are only regenerated, such as a processed special light image in which microvessels in the superficial layer and thick blood vessels in the middle and deep layers are selectively reproduced, can be generated.

In addition, by subtracting a processed special light image from the original special light image or another processed special light image obtained by processing with another filter, still another processed special light image in which a specific site is selectively removed from the original special light image, such as an image obtained by removing blood vessels in the middle and deep layers from the original special light image or an image obtained by removing microvessels in the superficial layer, can be generated.

That is, by subjecting the original special light image (captured special light observation image) to frequency processing, a processed special light image in which desired sites such as microvessels in the superficial layer of the mucous membrane and thick blood vessels in the middle and deep layers of the mucous membrane are extracted from the original special light image or unnecessary sites are removed therefrom can be generated.

Therefore, by compositing the processed special light image (special light observation image obtained after the frequency processing) used as an compositing image with the normal light observation image as will be described later, the normal light observation image can have the processed special light image obtained by extracting and/or removing desired sites from the original special light image.

Accordingly, the invention is capable of acquiring a special light observation image and a normal light observation image captured simultaneously or substantially simultaneously to generate an image making full use of the characteristics of the normal light observation image and the special light observation image, the image being obtained by providing an image of a remarkable site captured in the special light observation to the normal light image serving as the base which is light and in which the structure and state of the whole image region including the depth can be easily recognized.

The compositing image generating section 82c is not limited to the one which includes all of the LPF, BPF and HPF and may have appropriately selected one or more of them. However, the compositing image generating section 82c preferably has at least two filters to enable microvessels in the superficial layer and blood vessels in the middle and deep layers to be enhanced, removed or suppressed. A plurality of types of filters having different filtering properties such as a plurality of type of LPFs having different passbands may be included.

In the practice of the invention, processing performed to extract or remove a site of interest from the original special light image is not limited to frequency processing.

As is well known, light that entered the body tissue (observation light) diffusely propagates in the body tissue but the light absorption/scattering properties of the body tissue are wavelength-dependent, and light at shorter wavelengths tends to increase the scattering properties, that is, the invasion depth of light decreases. Therefore, microvessels in the superficial layer are imaged with narrowband B light, whereas thick blood vessels in the middle and deep layers are imaged with G light (narrowband G light). Therefore, in the special light observation image, the site in the superficial layer is bluish and thick blood vessels in the middle and deep layers are greenish.

The invention may use this characteristic to extract color components such as green and blue components from the original special light image to thereby generate a processed special light image in which microvessels in the superficial layer or thick blood vessels in the middle and deep layers are extracted or the like.

In the practice of the invention, image contrast is also available as processing for extracting or removing a site of interest from the original special light image.

In other words, the site having microvessels in the superficial layer or blood vessels in the middle and deep layers has higher contrast than on the periphery. This invention uses this characteristic to extract a portion (pixel) having higher contrast than a predetermined value to generate a processed special light image in which the microvessels in the superficial layer or the thick blood vessels in the middle and deep layers are extracted or the like.

In this process, the contrast threshold for the extraction may be appropriately set according to the site to be observed or the characteristics of the endoscope 12 based on the past experience and simulation.

In a portion having a cancer lesion, microvessels in the superficial layer may often have a predetermined shape in accordance with the disease progression (severity) or the site.

This characteristic may be used to store the shape (structure) of a blood vessel specific to the severity or the site of a disease beforehand and extract a portion having a predetermined shape from the original special light image by pattern matching, thereby generating a processed special light image in which a region which is more likely to be a lesion is preferentially extracted from the microvessels in the superficial layer and thick blood vessels in the middle and deep layers.

In this process, pattern matching (form recognition) may be performed by any known method. The blood vessel shape according to the severity of a disease or the like may be selected from those described in various documents or acquired by sampling images having clear lesions thereon.

In the practice of the invention, only one of frequency processing, color component extraction, contrast extraction and shape extraction may be performed, or two or more of the processing steps may be appropriately selected and used in combination.

A processed special light image may further be obtained by performing the processing steps such as frequency processing, color component extraction, contrast extraction and shape extraction, checking the processed image for the degree of denseness of the design, and further extracting a region where the degree of denseness exceeds a predetermined value.

Microvessels in the superficial layer tend to be denser in a portion having a lesion of cancer or the like than in a normal portion. Therefore, a processed special light image in which a region which is more likely to be a lesion is preferentially extracted may be generated by checking for the degree of denseness the image in which microvessels or the like are extracted by processing such as frequency processing and color component extraction and by further extracting the region having a high degree of denseness.

The degree of denseness on the image may be determined by known methods such as measurement of spatial frequency and measurement of MTF (Modulation Transfer Function). The threshold of the degree of denseness on which the extraction to be performed is based may be appropriately set according to the site to be observed or the characteristics of the endoscope 12 based on the past experience and simulation.

The image processing performed to generate a compositing image in the compositing image generating section 82c is not limited to frequency processing and extraction or removal of a specific site such as color component extraction, contrast extraction, shape extraction and extraction based on the degree of denseness. In other words, various known image processing steps which are performed on various images including endoscopic images may be optionally performed instead of such extraction or removal of a specific site to generate a compositing image.

For example when a site where the visibility was to be improved was extracted from the original special light image, the specific site of the processed special light image may be enhanced by sharpening, edge enhancement, an increase in the density or saturation of the image or differentiation from the peripheral site (image) through hue changes.

When a site which was not removed from the original special light image but was deemed to hinder the diagnosis was extracted, the specific site of the processed special light image may be suppressed by a decrease in the density or saturation or sign inversion (inversion of positive signals to negative signals).

As described above, microvessels in the superficial layer tend to be denser in a lesion of cancer or the like than in a normal portion.

Therefore, the processed special light image may also be checked for the degree of denseness so that a region where the degree of denseness exceeds a predetermined value is enhanced for the enhancement of a lesion or a region where the degree of denseness is up to the predetermined value is suppressed for the suppression of a normal portion.

In this process, only one or both of the enhancement and suppression may be performed. The enhancement of a region where the degree of denseness exceeds the predetermined value and/or the suppression of a region where the degree of denseness is up to the predetermined value may be selectable.

As described above, in the image captured with special light, the microvessels in the superficial layer is bluish and the blood vessels in the middle and deep layers are greenish. In other words, the special light observation image has different colors from those of the actual body and may provide a feeling of unnaturalness or strangeness.

The colors of the processed special light image may be accordingly changed to match with those of the actual body to thereby form an image having original colors of the body. The processed special light image whose colors were changed may be further enhanced by sharpening, edge enhancement or the like.

Conversely, differences between the colors of the processed special light image and the original colors of the body may be enhanced in order to further facilitate the visibility of microvessels in the superficial layer and blood vessels in the middle and deep layers.

The timing of such enhancement and suppression is not particularly limited.

For example, in cases where a processed special light image in which microvessels in the superficial layer are extracted and a processed special light image in which blood vessels in the middle and deep layers are extracted are prepared and the two images are composited to generate a compositing image, the compositing image may be formed by enhancing the individual processed special light images before composition or by compositing the two processed special light images before enhancing.

In cases where a plurality of processed special light images having undergone enhancement are composited to generate a compositing image, the individual processed special light images do not need to undergo the same processing. For example when two processed special light images are composited to generate a compositing image, the same enhancement may be performed by changing the intensity or image processing method. Alternatively, only one of the processed special light images may be enhanced or another method is also possible in which one of them is enhanced whereas the other is suppressed.

Image processing in the compositing image generating section 82c may be performed in response to an instruction inputted from an indication means (selection means) provided in the input device 20 or optionally in the operating section 28 of the endoscope 12 to indicate the display / enhancement / suppression / removal of microvessels, display / enhancement / suppression / removal of blood vessels in the middle and deep layers, and the color conversion of a predetermined site.

The compositing image generating section 82c subjects the original special light image (original special light observation image) to predetermined processing to generate a compositing image and supplies it to the image composition unit 84.

The image composition unit 84 composites the compositing image supplied from the special light image processing unit 82 with the normal light observation image supplied from the normal light image generating unit 80 to obtain a composite image and supplies the display signal generating unit 86 with the composite image for displaying on the monitor 18.

The image composition method in the image composition unit 84 is not particularly limited and various known image composition methods may be used.

The display signal generating unit 86 subjects the composite image supplied from the image composition unit 84 to color space conversion, scaling and other necessary processing steps, or image allocation, incorporation of character information such as the name of a subject and other necessary processing steps to generate a display image having the composite image incorporated therein and this image is displayed on the monitor 18.

The display signal generating unit 86 may receive not only the composite image but also images from the normal light image generating unit 80 and the special light image processing unit 82 in response to an instruction given from the input device 20 or the indication means provided in the operating section 28 of the endoscope 12 so that the monitor 18 may display not only the composite image but also the normal light observation image and the special light observation image (original special light image and/or the processed special light image).

In this case, the monitor 18 may display all the images, two or more appropriately selected images, or one appropriately selected image. In addition, display modes such as three-image display, two-image display, and toggle image display are available.

An example of the operation of the endoscope system 10 is described below.

When the input device 20 issues an instruction for the start of imaging with the endoscope 12, the light source 60 of the light source device 16 is turned on, the rotary drive source 72 starts to rotate the rotary filter 64, and the CCD sensor 48 starts imaging (photometry) in synchronization with the rotation of the rotary filter 64.

Light emitted from the light source 60 is collimated by the collimator lens 62, separated by the rotary filter 64 in a time sharing manner into R light, G light, B light and narrowband B light (and optionally narrowband G light), and enters the optical fiber 70 through the condenser lens 68.

The optical fiber 70 propagates the incident light which is supplied through the connecting portion 16a to the connector 32 of the endoscope 12 as observation light.

The observation light supplied to the connector 32 of the endoscope 12 propagates through the optical fiber 52 to the scope portion 42, where the observation light is emitted from the distal end of the optical fiber 52 to be irradiated through the illumination lens 50 on the observation site in the living body.

The observation site having received the observation light is imaged through the imaging lens 46 on the light-receiving surface of the CCD sensor 48, which captures an R image, a G image, a B image and a narrowband B image (and optionally a narrowband G image) in a frame sequential manner (performs photometry).

Output signals from the CCD sensor 48 are supplied to the AFE board 56. The AFE board 56 subjects the output signals from the CCD sensor 48 to noise removal by correlated double sampling, amplification and A/D conversion to obtain digital image signals, which are then supplied to the DSP 76 of the processing device 14 (processor 14a).

The DSP 76 subjects the supplied image (image signals) to predetermined processing such as gamma correction and color correction and stores the processed image in a predetermined portion of the storage unit 78.

Once the image signals are stored in the storage unit 78, the read-out section 80a of the normal light image generating unit 80 reads out the image signals of the R, G and B images from the storage unit 78 and supplies them to the image processing section 80b. The read-out section 82a of the special light image processing unit 82 reads out the narrowband B image and (narrowband) G image from the storage unit 78 and supplies them to the image processing section 82b.

In the normal light image generating unit 80, the image processing section 80b performs color conversion, color enhancement and image structure enhancement and supplies the resulting normal light observation image to the image composition unit 84.

On the other hand, in the special light image processing unit 82, the image processing section 82b allocates the G image to R pixels to be displayed and the narrowband B image to B and G pixels to be displayed to form pixels each composed of three sub-pixels, and the image is further subjected to color conversion, color enhancement and image structure enhancement. The resulting image is supplied to the compositing image generating section 82c as the special light observation image (original special light image).

The compositing image generating section 82c performs frequency processing on the original special light image supplied thereto to generate a compositing image.

For example, the original special light image is processed with an HPF to generate a processed special light image in which surface microvessels are extracted (i.e., thick blood vessels in the middle and deep layers are removed) as a compositing image.

Alternatively, the original special light image is processed with an HPF to generate a first processed special light image in which surface microvessels are extracted and is also processed with an LPF to generate a second processed special light image in which blood vessels in the middle and deep layers are extracted). Then, the first and second processed special light images are subjected to enhancement processing and suppression processing, respectively. Then, the first processed special light image is composited with the second processed special light image to generate as a compositing image a processed special light image in which the surface microvessels are enhanced but the thick blood vessels in the middle and deep layers are suppressed.

Once the compositing image is generated, the compositing image generating section 82c supplies the thus generated compositing image to the image composition unit 84.

The image composition unit 84 composites the normal light observation image supplied from the normal light image generating unit 80 with the compositing image supplied from the special light image processing unit 82 to generate a composite image in which information of the special light image is provided to the normal light observation image, and supplies the composite image to the display signal generating unit 86.

The display signal generating unit 86 generates a display image in which the composite image supplied thereto is incorporated and the monitor 18 displays the display image.

In the foregoing embodiment, the image processing device of the invention is applied to the endoscope system 10 which includes the light source device 16 using the rotary filter 64 with the filters for R light, G light, B light and narrowband B light, and the endoscope 12 using the monochrome CCD sensor 48. However, this is not the sole case of the invention.

That is, the image processing device of the invention (the illustrated processing device 14) can be applied to various endoscope systems which use a light source device and an endoscope capable of capturing images used for a normal light observation image and a special light observation image simultaneously or substantially simultaneously.

For example, in the endoscope system 10 shown in FIGS. 1 and 2, another configuration is possible in which the light source device 16 has no rotary filter and the monochrome CCD sensor 48 used as the CCD sensor of the endoscope 12 is replaced by a four-color (five-color) CCD sensor which separates incident light into R light, G light, B light and narrowband B light (and optionally narrow-bang G light) for photometry, thereby capturing an R image, a G image, a B image and a narrowband B image and obtaining simultaneously captured normal light observation image and special light observation image as in the above embodiment.

In the endoscope system 10 shown in FIGS. 1 and 2, still another configuration is possible in which the rotary filter 64 of the light source device 16 is replaced by a rotary filter which has in its rotational direction a colorless transparent filter or a hole and a narrowband B filter converting white light into narrowband B light, and the monochrome CCD sensor 48 of the endoscope 12 is replaced by a common color CCD sensor.

The common color CCD sensor is a sensor which is used in a common endoscope or digital camera and separates incident light into R light, G light and B light of three primary colors for photometry.

According to this configuration, the light source device rotates the rotary filter to alternately supply to the endoscope white light having passed through the colorless transparent filter and narrowband B light having passed through the narrowband B filter. In synchronization with the rotation of the rotary filter, the color CCD sensor of the endoscope alternately captures a normal light observation image including an R image, a G image and a B image and using white light as observation light and a narrowband B image using narrowband B light as observation light.

The storage unit 78 of the processing device 14 thus stores the simultaneously captured R, G, B and narrowband B images as in the foregoing embodiment.

Therefore, the normal light image generating unit 80 and the special light image processing unit 82 likewise read out the R, G and B images, and the narrowband B image and G image from images stored in the storage unit 78, respectively, thereby obtaining simultaneously (substantially simultaneously) captured normal light observation image and special light observation image.

In this configuration as well, the rotary filter may further include a narrowband G filter in addition to the colorless transparent filter and the narrowband B filter so that a special light observation image is also generated from the narrowband B image and the narrowband G image.

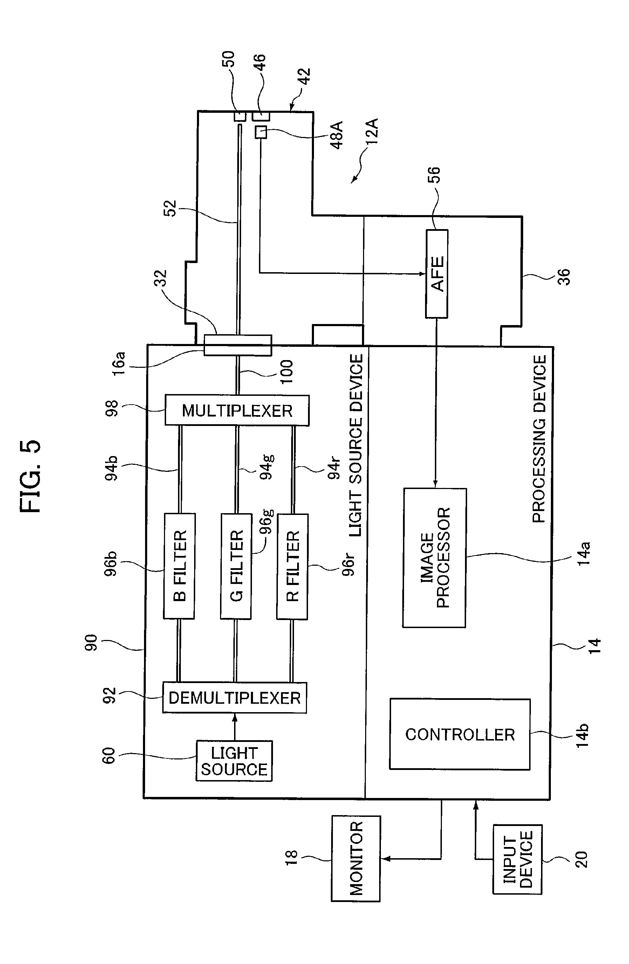

The configuration using a light source device 90 as shown in FIG. 5 is another example which also uses an endoscope having a common color CCD sensor.

In FIG. 5, like components are denoted by the same reference numerals as in FIG. 2 and the following description mainly focuses on the different features. In an endoscope shown by reference numeral 12A, a CCD sensor 48A is the foregoing common color CCD sensor.

The light source device 90 includes the light source 60 which is the same as that used in the above embodiment, a demultiplexer 92, a B fiber 94b, a G fiber 94g, an R fiber 94r, a B filter 96b, a G filter 96g, an R filter 96r, a multiplexer 98, an optical fiber 100 and the connecting portion 16a which is the same as that used in the above embodiment.

In the illustrated light source device 90, white light emitted from the light source 60 is separated by the demultiplexer 92 into three components, which enter the optical fibers including the B fiber 94b, G fiber 94g and R fiber 94r and are propagated therethrough to the multiplexer 98.

The B filter 96b, the G filter 96g and the R filter 96r are provided between the demultiplexer 92 and the multiplexer 98 in the B fiber 94b, G fiber 94g and R fiber 94r, respectively. Entry of light in the filters from the optical fibers and exit of light from the filters to the optical fibers may be performed by any known method.

The B filter 96b is a filter for converting white light into narrowband B light. The G filter 96g is a filter for converting white light into narrowband G light or G light. The R filter 96r is a filter for converting white light into R light.

Therefore, the light propagated through the B fiber 94b is converted by the B filter 96b into narrowband B light, the light propagated through the G fiber 94g is converted by the G filter 96g into narrowband G light, and the light propagated through the R fiber 94r is converted by the R filter 96r into R light. The narrowband B light, the narrowband G light and the R light then enter the multiplexer 98.

The narrowband B light, the narrowband G light and the R light are converged in the multiplexer 98, propagated through the optical fiber 100 and supplied through the connecting portion 16a to the endoscope 12A.

In other words, the endoscope is supplied with pseudo white light which is a mixture of the narrowband B light, the narrowband G light and the R light and captures images using the pseudo white light as the observation light.

As described above, the CCD sensor of the endoscope is a color CCD sensor. Therefore, an R image, a G image and a B image are outputted from the endoscope and stored in the storage unit 78 of the processing device 14.

The observation light used is pseudo white light which is a mixture of the narrowband B light, the narrowband G light and the R light, and the CCD sensor 48A is a color CCD sensor. Therefore, the B image, G image and R image captured by the CCD sensor 48A can be used to generate a normal light observation image which uses white light as the observation light.

The B light and G light used for the observation are both narrowband. Therefore, the B image and G image captured by the CCD sensor 48A can be used to generate a special light observation image which uses the narrowband B light and narrowband G light as the observation light.

Therefore, the normal light image generating unit 80 and the special light image processing unit 82 read out the R, G and B images and the B and G images from images stored in the storage unit 78, respectively, whereby simultaneously captured normal light observation image and special light observation image can be obtained.

In the light source device 90 shown in FIG. 5, it is preferred to provide between the filters and the multiplexer 98 light quantity adjusting means which are each independently capable of adjusting the quantity of light and to supply each light to the multiplexer 98 so that a relation of B light > G light > R light is met for the quantity (intensity) of light.

In a common color CCD sensor, pixels of B, G and R colors have sensitivity until the neighboring color area owing to the filtering characteristics of the B, G and R colors. In other words, G pixels allow for the incidence and photometry of the narrowband G light and R-band light, and B pixels allow for the incidence and photometry of the narrowband B light and narrowband G light.

In contrast, by adjusting the quantity of light so that the relation of B light > G light > R light is met, the narrowband B light may predominantly enter the B pixels of the CCD sensor and the narrowband G light may likewise predominantly enter the G pixels. A proper normal light observation image and a proper special light observation image can be thus generated from the images read by the CCD sensor.

In cases where the quantity of light used for the observation meets the relation of B light > G light > R light, the images are preferably first subjected to gain adjustment in the normal light image generating unit 80 to obtain the same images as those captured with white observation light in which the B light, G light and R light are equal in quantity.

Amplification of the G image and the R image and reduction of the B image and the G image may be performed by gain adjustment using, for example, multiplication by a correction coefficient or processing with an LUT so as to obtain the same images as those captured with white observation light in which the B light, G light and R light are equal in quantity.

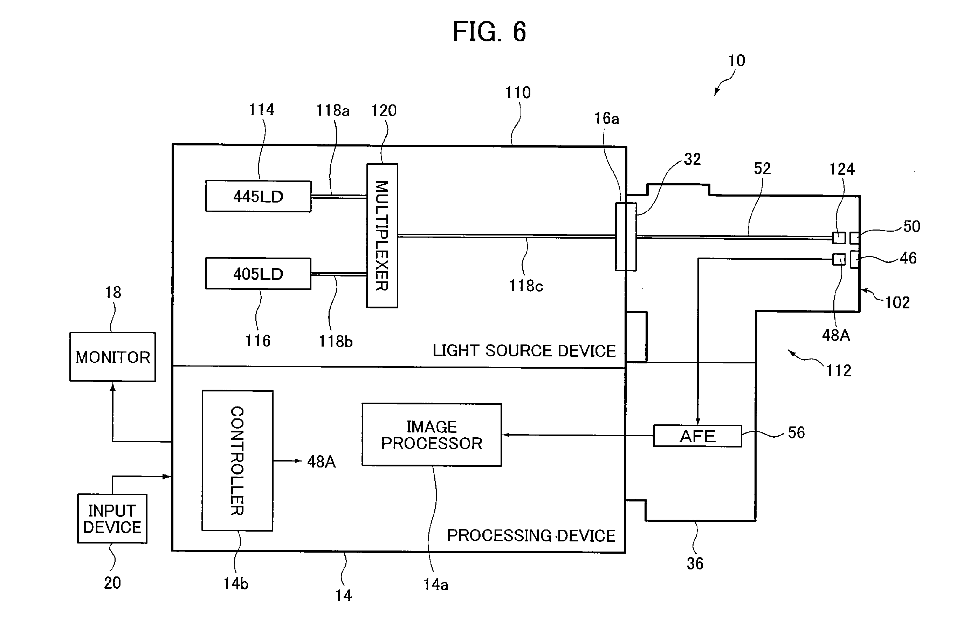

The configuration using a light source device 110 and an endoscope 112 as shown in FIG. 6 is still another example which also uses an endoscope having a common color CCD sensor.

In FIG. 6, like components are denoted by the same reference numerals as in FIG. 5 and the following description mainly focuses on the different features.

The light source device 110 includes a 445LD 114, a 405LD 116, optical fibers 118a, 118b and 118c, and a multiplexer 120.

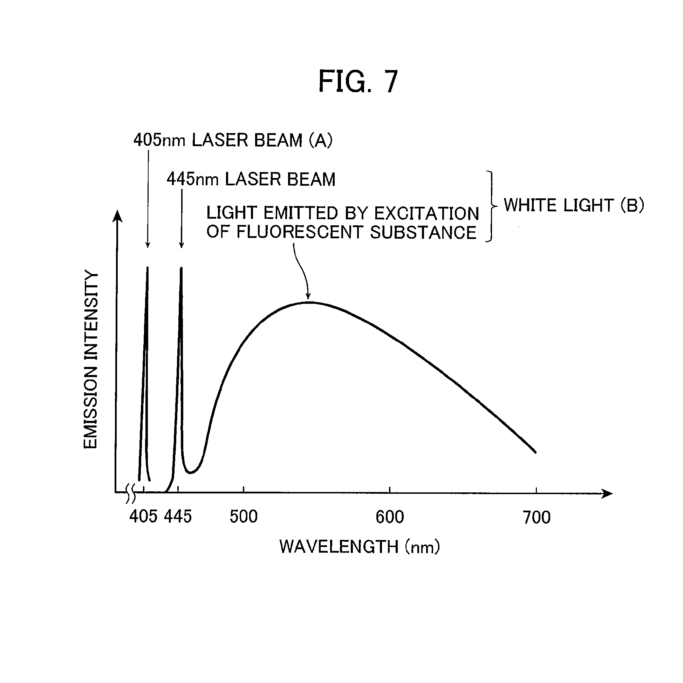

The 445LD 114 is a light source emitting B laser beams with a central wavelength of 445 nm having the spectrometric profile shown in FIG. 7. On the other hand, the 405LD 116 is a light source emitting narrowband blue-violet (V) laser beams with a central wavelength of 405 nm having the spectrometric profile shown in FIG. 7.

The B light emitted from the 445LD 114 is propagated through the optical fiber 118a and the V light emitted from the 405LD 116 is propagated through the optical fiber 118b and the B light and V light are multiplexed into a single beam by the multiplexer 120.

The B light and V light are multiplexed into the single beam, which is propagated through the optical fiber 118c, is supplied through the connecting portion 16a to the connector 32 of the endoscope 112, and enters and is propagated through the optical fiber 52 to exit from the distal end of the optical fiber 52.

In the endoscope 112, a fluorescent substance 124 is disposed at the distal end of the optical fiber 52. The fluorescent substance 124 includes a plurality of kinds of phosphors (e.g., YAG phosphor and BAM (BaMgAl₁₀O₁₇) phosphor) which absorb part of B light, excite and emit green to yellow light. The green to yellow light emitted by excitation of the fluorescent substance having absorbed B light as excitation light is combined with B light which was not absorbed in the fluorescent substance 124 but passed therethrough to form pseudo white light.

Most of the V light (V laser beams) emitted from the 405LD 116 passes through the fluorescent substance 124 without being absorbed therein.

FIG. 7 shows emission spectral characteristics of the V light emitted from the 405LD 116, the B light emitted from the 445LD 114 and the light emitted from the fluorescent substance 124 excited by the B light.

As shown in FIG. 7, the V light emitted from the 405LD 116 is narrowband V light represented by an emission line with a central wavelength of 405 nm (profile A).

The B light emitted from the 445LD 114 is represented by an emission line with a central wavelength of 445 nm. In addition, the light emitted from the fluorescent substance 124 excited by the B light has a spectral intensity distribution which shows an increase in emission intensity in a wavelength range of about 450 nm to about 700 nm.

Therefore, when only the 445LD 114 is turned on in the light source device 110, the light emitted by the excitation of the fluorescent substance 124 is combined with the B light from the 445LD 114 which was not absorbed in the fluorescent substance 124 to form pseudo white light (profile B), which enables normal light observation using white light as the observation light.

When both of the 445LD 114 and the 405LD 116 are turned on, narrowband V light shown by profile A is added to the observation light, thus enabling imaging with special light.

More specifically, the 405LD 116 in the light source device 110 is turned on and off in a predetermined period (or high power lighting and low power lighting are alternately repeated) under the control of the controller 14b so that images are captured by the CCD sensor 48A of the endoscope 112 in synchronization with the on/off status of the 405LD 116.

Accordingly, the normal light image generating unit 80 reads out the R, G and B images from images stored in the storage unit 78 when the 405LD 116 is turned off (at the time of low power lighting) and the special light image processing unit 82 reads out the B image and the G image from the images stored in the storage unit 78 when the 405LD 116 is turned on (at the time of high power lighting), thus enabling simultaneously (substantially simultaneously) captured normal light observation image and special light observation image to be obtained.

In this embodiment, the 405LD 116 is not turned on and off but is always turned on to generate a composite image so that simultaneously captured normal light observation image and special light observation image may be obtained as above.

While the image processing device of the invention has been described above in detail, the invention is by no means limited to the above embodiments, and various improvements and modifications may of course be made without departing from the spirit of the invention.

## Claims

1. An image processing device comprising:
an image acquisition unit for acquiring a normal light observation image captured by an endoscope using white light as observation light and an original special light observation image captured by the endoscope simultaneously with the normal light observation image using predetermined narrowband light as the observation light; and
an image processing unit for subjecting the original special light observation image acquired by the image acquisition unit to predetermined processing to generate a special light observation image and providing information of the special light observation image to the normal light observation image.

2. The image processing device according to claim 1, wherein the image processing unit performs frequency processing on the original special light observation image as the predetermined processing.

3. The image processing device according to claim 2, wherein the image processing unit has at least one function selected from a function of processing the original special light observation image with a high-pass filter, a function of processing the original special light observation image with a band-pass filter, and a function of processing the original special light observation image with a low-pass filter.

4. The image processing device according to claim 2 or 3,
wherein the image processing device further comprises a filter selector for selecting a filter to be used in the frequency processing of the original special light observation image in the image processing unit, and
wherein the image processing unit has different filters for use in the frequency processing of the original special light observation image and a filter selected by the filter selector is used to perform the frequency processing on the original special light observation image.

5. The image processing device according to claim 4,
wherein the image processing unit prepares a first image obtained by performing the frequency processing on the original special light observation image with a first filter and a second image obtained by performing the frequency processing on the original special light observation image with a different filter from the first filter, and information of an image obtained by compositing the first image with the second image or information obtained by subtracting one of the first image and the second image from the other is provided to the normal light observation image.

6. The image processing device according to any one of claims 1 to 5, wherein the image processing unit extracts a predetermined color component from the original special light observation image as the predetermined processing.

7. The image processing device according to any one of claims 1 to 6, wherein the image processing unit extracts a portion having a predetermined contrast from the original special light observation image as the predetermined processing.

8. The image processing device according to any one of claims 1 to 7, wherein the image processing unit extracts a portion having a predetermined structure as the predetermined processing.

9. The image processing device according to any one of claims 1 to 8, wherein the image processing unit checks the special light observation image after the predetermined processing for a degree of denseness and further extracts a region where the degree of denseness exceeds a predetermined value.

10. The image processing device according to any one of claims 1 to 9,
wherein the image processing device further comprises a processing selector for selecting enhancement or suppression of the special light observation image generated by processing the original special light observation image, and
wherein the image processing unit enhances or suppresses the special light observation image generated by processing the original special light observation image in accordance with a selection made by the processing selector.

11. The image processing device according to any one of claims 1 to 10, wherein the image processing unit checks the special light observation image after the predetermined processing for a degree of denseness and performs at least one processing selected from enhancement of a region where the degree of denseness exceeds a predetermined value and suppression of a region where the degree of denseness is up to the predetermined value.
